(19) Europäisches Patentamt
European Patent Office
Office européen des brevets

(11) Publication number: 0 094 245 A2

(12) # EUROPEAN PATENT APPLICATION

(21) Application number: 83302632.1

(22) Date of filing: 10.05.83

(51) Int. Cl.³: C 07 D 471/04, A 61 K 31/44, A 61 K 31/495, A 61 K 31/535 // (C07D471/04, 221/00, 221/00)

(30) Priority: 10.05.82 JP 77794/82

(43) Date of publication of application: 16.11.83 Bulletin 83/46

(84) Designated Contracting States: BE CH DE FR GB IT LI NL

(71) Applicant: SANKYO COMPANY LIMITED, No. 1-6, 3-chome Nihonbashi Honcho Chuo-ku, Tokyo (JP)

(72) Inventor: Terada, Atsusuke Chemical Research Laboratories, Sankyo Company Limited, 2-58, 1-chome, Hiromachi Shinagwa-ku Tokyo (JP)
Inventor: Hattori, Chiharu Chemical Research Laboratories, Sankyo Company Limited, 2-58, 1-chome, Hiromachi Shinagwa-ku Tokyo (JP)
Inventor: Misaka, Eiichi c/o Biological Research Lab., Sankyo Company Limited, 2-58, 1-chome, Hiromachi Shinagwa-ku Tokyo (JP)

(74) Representative: Tubby, David George et al, MARKS & CLERK 57-60 Lincoln's Inn Fields, London WC2A 3LS (GB)

(54) **Dihydronaphthyridine derivatives, their preparation and compositions containing them.**

(57) Compounds of formula (I)

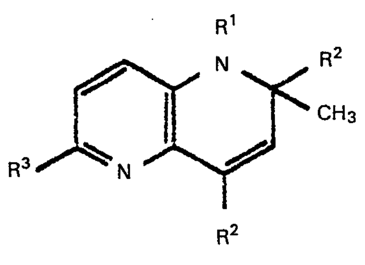

(I)

(wherein $R^1$ represents a hydrogen atom or an alkyl or acyl group, $R^2$ represents an alkyl group and $R^3$ represents a hydrogen or halogen atom or an alkyl, hydroxy, alkoxy, aryloxy, optionally substituted pyridyloxy, alkylthio, arylthio, amino or mono- or di-substituted amino group) and pharmaceutically acceptable acid addition salts thereof have useful analgesic and anti-inflammatory properties. They may be prepared by condensing a 5-amino-2-($R^3$)pyridine with a ketone of formula $R^2COCH_3$, or with a condensation product of said ketone, to give a compound of formula (I) in which $R^1$ represents a hydrogen atom and then, if desired, alkylating or acylating that compound.

M&C FOLIO: 230P45967 WANGDOC: 0151C

# DIHYDRONAPHTHYRIDINE DERIVATIVES, THEIR PREPARATION AND COMPOSITIONS CONTAINING THEM

The present invention relates to a series of new dihydronaphthyridine derivatives which have valuable analgesic and anti-inflammatory activities; the invention also provides processes for preparing these derivatives and pharmaceutical compositions containing them.

Mild analgesics and anti-inflammatory agents are amongst the most commonly used of drugs. Most such drugs in common use have side effects which may be distressing or even dangerous to a small percentage of the population - even though the number of people so afflicted may be statistically insignificant, it is better for such persons to employ a different analgesic or anti-inflammatory drug, whose side effects may not be distressing or dangerous to them, rather than to continue with the original drug. There is, therefore, a continuing need for new analgesic and anti-inflammatory drugs, to broaden the choice available to the user.

We have now discovered a series of new dihydronaphthyridine derivatives which have excellent analgesic and anti-inflammatory activities and which, in addition, inhibit peroxidation of lipids.

The compounds of the invention may be represented by the formula (I):

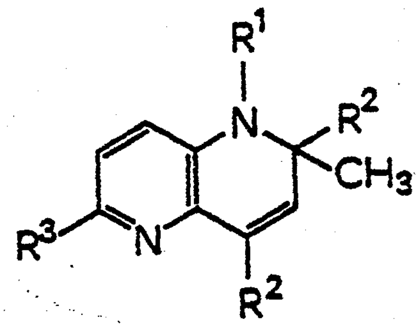

(I)

(in which:

$R^1$ represents a hydrogen atom, an alkyl group or an acyl group;

$R^2$ represents an alkyl group, and the two groups represented by $R^2$ may be the same or different; and

$R^3$ represents a hydrogen atom, a halogen atom, an alkyl group, a hydroxy group, an alkoxy group, an aryloxy group, a pyridyloxy group, a pyridyloxy group having at least one substituent in its pyridine ring, an alkylthio group, an arylthio group, an amino group or a mono-or di-substituted amino group)

and pharmaceutically acceptable acid addition salts thereof.

The invention also provides a process for preparing compounds of formula (I) and their salts, which

comprises:

(a) reacting a compound of formula (II):

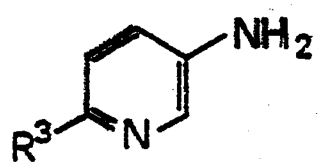

(II)

(in which $R^3$ is as defined above) with a compound of formula (III), (IV) or (V):

$R^2COCH_3$ (III)

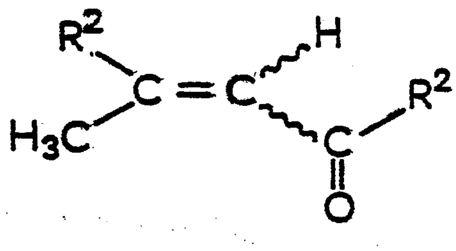

(IV)

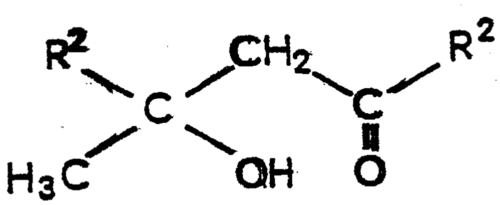

(V)

(in which $R^2$ is as defined above), to give a compound of formula I(a):

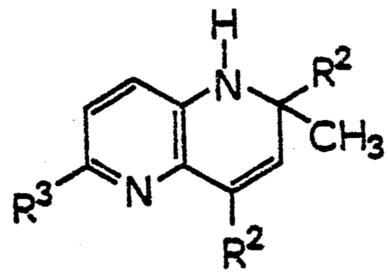

(Ia)

(in which $R^2$ and $R^3$ are as defined above), that is to say a compound of formula (I) in which $R^1$ represents a hydrogen atom; and

(b) optionally, reacting said compound of formula I(a) with a compound of formula (VI):

$$R^4X \quad (VI)$$

(in which:

$R^4$ represents an alkyl group and X represents a halogen atom; or

$R^4$ represents an acyl group and X represents a halogen atom or an acyloxy group), to give a compound of formula I(b):

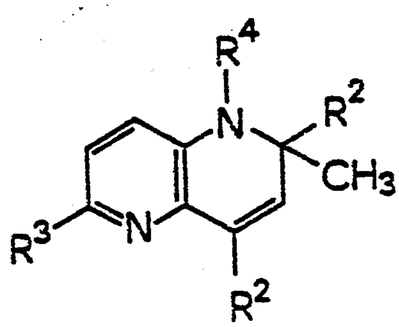

(Ib)

(in which $R^2$, $R^3$ and $R^4$ are as defined above), that is to say a compound of formula (I) in which $R^1$ represents an alkyl group or an acyl group; and

(c) optionally salifying the product of step (a) or (b).

The invention also provides a pharmaceutical composition for analgesic or anti-inflammatory use and comprising, as active ingredient, at least one compound of the present invention in admixture with a pharmaceutically acceptable carrier or diluent.

Where $R^1$ or $R^4$ represents an alkyl group, this may be a straight or branched chain alkyl group and is preferably such a group having from 1 to 6 carbon atoms. Examples of such groups include the methyl, ethyl, propyl, isopropyl, butyl, isobutyl, pentyl and hexyl groups.

Where $R^1$ or $R^4$ represents an acyl group, this is preferably: an aliphatic acyl group having from 1 to 4 carbon atoms, for example an acetyl, propionyl or butyryl group; an aromatic acyl group, for example a benzoyl or naphthoyl group, which may be unsubstituted or which may have one or more substituents in its aromatic ring, said substitutents being, for example, halogen atoms (such as fluorine, chlorine or bromine atoms), $C_1$-$C_6$ alkyl groups (such as methyl, ethyl or propyl groups) or $C_1$-$C_6$ alkoxy groups (such as methoxy, ethoxy or propoxy groups); or an araliphatic

acyl group, such as a phenylacetyl or 2-phenylpropionyl group.

$R^2$ preferably represents an alkyl group having from 1 to 6 carbon atoms, more preferably a methyl, ethyl, propyl, isopropyl, butyl, isobutyl, pentyl or hexyl group and most preferably a $C_1$-$C_3$ alkyl group, e.g. methyl, ethyl or propyl, particularly methyl. The two groups represented by $R^2$ may be the same or different, but, for convenience of preparation, are usually the same.

Where $R^3$ represents an alkyl group, this may be a straight or branched chain group and is preferably such a group having from 1 to 6 carbon atoms, more preferably a methyl, ethyl, propyl, isopropyl, butyl, isobutyl, pentyl or hexyl group and most preferably a $C_1$-$C_3$ alkyl group, e.g. methyl, ethyl or propyl.

Where $R^3$ represents an alkoxy group, this may be a straight or branched chain group and is preferably such a group having from 1 to 8 carbon atoms, more preferably a methoxy, ethoxy, propoxy, isopropoxy, butoxy, isobutoxy, t-butoxy, pentyloxy, hexyloxy, heptyloxy, octyloxy or 2-ethylhexyloxy group, most preferably a $C_1$-$C_3$ alkoxy group, e.g. methoxy, ethoxy, propoxy or isopropoxy.

Where $R^3$ represents an aryloxy or arylthio group, this is preferably a phenoxy or phenylthio group, which may be unsubstituted or may have one or more substituents on the benzene ring. Where $R^3$ represents a pyridyloxy group, this may be unsubstituted or may have one or more substituents on the pyridine ring. In the case of the substituted phenoxy, phenylthio or pyridyloxy groups, examples of preferred substituents include: halogen atoms, such as fluorine, chlorine or bromine atoms; the trifluoromethyl group; $C_1$-$C_6$ alkyl groups, such as methyl, ethyl or propyl groups, $C_1$-$C_6$ alkoxy groups, such as methoxy, ethoxy or propoxy groups, di($C_1$-$C_6$ alkyl)amino groups, such as dimethylamino, diethylamino or diisopropylamino groups; carboxy groups; ($C_1$-$C_6$ alkoxy)carbonyl groups, such as methoxycarbonyl or ethoxycarbonyl groups; or ($C_1$-$C_6$ alkoxy)-carbonyl($C_1$-$C_6$ alkyl) groups, such as methoxycarbonylmethyl, ethoxycarbonylmethyl or 2-methoxycarbonylethyl groups.

Where $R^3$ represents an alkylthio group, it may be a straight or branched chain group and is preferably such a group having from 1 to 6 carbon atoms, for example a methylthio, ethylthio, propylthio or isopropylthio group.

Where $R^3$ represents a mono-substituted amino group, it is preferably such a group having the formula

$-NHR^5$, in which $R^5$ represents: a $C_1$-$C_6$ alkyl group, for example a methyl, ethyl, propyl or isopropyl group; or a phenyl or pyridyl group, which may be unsubstituted or may have one or more substituents on its aromatic ring, said substituents being, for example, $C_1$-$C_6$ alkyl groups (especially methyl or ethyl groups), $C_1$-$C_6$ alkoxy groups (especially methoxy or ethoxy groups), hydroxy groups or halogen atoms (especially fluorine, chlorine or bromine atoms).

Where $R^3$ represents a di-substituted amino group, it is preferably a group of formula $-NR^6R^7$, in which $R^6$ and $R^7$ are the same or different and each represents an alkyl group (for example a methyl, ethyl or propyl group) or, together with the nitrogen atom to which they are attached, form a heterocyclic amino group, such as a 1-pyrrolidinyl group, a piperidinyl group, a morpholino group, a 1-piperazinyl group or a 1-piperazinyl group having in its 4-position at least one substituent which is a $C_1$-$C_6$ alkyl group (preferably methyl or ethyl), an aliphatic acyl group (preferably acetyl or propionyl), an aromatic acyl group (preferably benzoyl, p-chlorobenzoyl or o-methoxybenzoyl) or an aralkyl group (preferably benzyl, phenacyl, p-aminobenzyl or o-methoxyphenacyl).

Examples of the compounds of the present invention are given in the following list; the numbers assigned to

the compounds in this list are hereinafter used, where appropriate, to refer to the compounds:

1. 6-methoxy-2,2,4-trimethyl-1,2-dihydro-1,5-naphthyridine;
2. 6-ethoxy-2,2,4-trimethyl-1,2-dihydro-1,5-naphthyridine;
3. 2,2,4-trimethyl-6-propoxy-1,2-dihydro-1,5-naphthyridine;
4. 6-butoxy-2,2,4-trimethyl-1,2-dihydro-1,5-naphthyridine;
5. 2,2,4-trimethyl-6-phenoxy-1,2-dihydro-1,5-naphthyridine;
6. 6-(4-methoxyphenoxy)-2,2,4-trimethyl-1,2-dihydro-1,5-naphthyridine;
7. 2,2,4-trimethyl-6-p-tolyloxy-1,2-dihydro-1,5-naphthyridine;
8. 2,2,4-trimethyl-6-(3-trifluoromethylphenoxy)-1,2-dihydro-1,5-naphthyridine;
9. 6-(3-chlorophenoxy)-2,2,4-trimethyl-1,2-dihydro-1,5-naphthyridine;
10. 6-(2-chlorophenoxy)-2,2,4-trimethyl-1,2-dihydro-1,5-naphthyridine;
11. 6-(4-ethoxycarbonylphenoxy)-2,2,4-trimethyl-1,2-dihydro-1,5-naphthyridine;
12. 6-(4-carboxyphenoxy)-2,2,4-trimethyl-1,2-dihydro-1,5-naphthyridine;

13. 6-chloro-2,2,4-trimethyl-1,2-dihydro-1,5-naphthyridine;

14. 2,2,4-trimethyl-6-phenylthio-1,2-dihydro-1,5-naphthyridine;

15. 2,2,4-trimethyl-6-p-tolylthio-1,2-dihydro-1,5-naphthyridine;

16. 2,2,4-trimethyl-6-o-tolylthio-1,2-dihydro-1,5-naphthyridine;

17. 6-(4-chlorophenylthio)-2,2,4-trimethyl-1,2-dihydro-1,5-naphthyridine;

18. 2,2,4-trimethyl-6-(3-pyridyloxy)-1,2-dihydro-1,5-naphthyridine;

19. 2,2,4-trimethyl-6-morpholino-1,2-dihydro-1,5-naphthyridine;

20. 2,2,4-trimethyl-6-piperidino-1,2-dihydro-1,5-naphthyridine;

21. 6-diethylamino-2,2,4-trimethyl-1,2-dihydro-1,5-naphthyridine;

22. 6-dimethylamino-2,2,4-trimethyl-1,2-dihydro-1,5-naphthyridine;

23. 2,2,4-trimethyl-6-(1-pyrrolidinyl)-1,2-dihydro-1,5-naphthyridine;

24. 6-hydroxy-2,2,4-trimethyl-1,2-dihydro-1,5-naphthyridine;

25. 6-methoxy-1,2,2,4-tetramethyl-1,2-dihydro-1,5-naphthyridine;

26. 6-chloro-1,2,2,4-tetramethyl-1,2-dinydro-1,5-naphthyridine;

27. 1-acetyl-6-methoxy-2,2,4-trimethyl-1,2-dihydro-1,5-naphthyridine;

28. 1-benzoyl-6-methoxy-2,2,4-trimethyl-1,2-dihydro-1,5-naphthyridine;

29. 1-cinnamoyl-6-methoxy-2,2,4-trimethyl-1,2-dihydro-1,5-naphthyridine;

30. 2,4-diethyl-6-methoxy-2-methyl-1,2-dihydro-1,5-naphthyridine;

31. 6-chloro-2,4-diethyl-2-methyl-1,2-dihydro-1,5-naphthyridine;

32. 2,2,4-trimethyl-6-(4-pyridyloxy)-1,2-dihydro-1,5-naphthyridine;

33. 2,2,4-trimethyl-6-(2-pyridyloxy)-1,2-dihydro-1,5-naphthyridine;

34. 2,2,4-trimethyl-6-(6-methyl-3-pyridyloxy)-1,2-dihydro-1,5-naphthyridine;

35. 2,2,4-trimethyl-6-(6-methyl-2-pyridyloxy)-1,2-dihydro-1,5-naphthyridine;

36. 6-(2-chloro-3-pyridyloxy)-2,2,4-trimethyl-1,2-dihydro-1,5-naphthyridine;

37. 6-(5-chloro-2-pyridyloxy)-2,2,4-trimethyl-1,2-dihydro-1,5-naphthyridine;

38. 6-(5-chloro-3-pyridyloxy)-2,2,4-trimethyl-1,2-dihydro-1,5-naphthyridine;

39. 6-(2-bromo-3-pyridyloxy)-2,2,4-trimethyl-1,2-dihydro-1,5-naphthyridine;

40. 6-hexyloxy-2,2,4-trimethyl-1,2-dihydro-1,5-naphthyridine;

41. 2,2,4-trimethyl-6-octyloxy-1,2-dihydro-1,5-naphthyridine;

42. 6-(4-chlorophenoxy)-2,2,4-trimethyl-1,2-dihydro-1,5-naphthyridine;

43. 6-(4-ethoxycarbonylmethylphenoxy)-2,2,4-trimethyl-1,2-dihydro-1,5-naphthyridine;

44. 6-(2-ethoxycarbonylphenoxy)-2,2,4-trimethyl-1,2-dihydro-1,5-naphthyridine;

45. 6-(3-dimethylaminophenoxy)-2,2,4-trimethyl-1,2-dihydro-1,5-naphthyridine;

46. 6-benzyloxy-2,2,4-trimethyl-1,2-dihydro-1,5-naphthyridine;

47. 6-butoxy-1,2,2,4-tetramethyl-1,2-dihydro-1,5-naphthyridine;

48. 1-(p-chlorobenzoyl)-6-methoxy-2,2,4-trimethyl-1,2-dihydro-1,5-naphthyridine;

49. 1-(o-fluorobenzoyl)-6-methoxy-2,2,4-trimethyl-1,2-dihydro-1,5-naphthyridine;

50. 1-acetyl-2,2,4-trimethyl-6-morpholino-1,2-dihydro-1,5-naphthyridine;

51. 6-(3-methoxyphenylthio)-2,2,4-trimethyl-1,2-dihydro-1,5-naphthyridine;

52. 6-(4-methoxyphenylthio)-2,2,4-trimethyl-1,2-dihydro-1,5-naphthyridine;

53. 6-(3-chloro-5-methoxyphenoxy)-2,2,4-trimethyl-1,2-dihydro-1,5-naphthyridine;

54. 6-(3,4-dimethoxyphenoxy)-2,2,4-trimethyl-1,2-dihydro-1,5-naphthyridine;

55. 6-(3,4-dichlorophenoxy)-2,2,4-trimethyl-1,2-dihydro-1,5-naphthyridine;

56. 6-(3,5-dichlorophenoxy)-2,2,4-trimethyl-1,2-dihydro-1,5-naphthyridine.

Of the compounds listed above, Compounds No. 1, 2, 4, 5, 13, 18, 25 and 27 are preferred, Compounds No. 1 and 18 being most preferred.

The compounds of the invention may be prepared by the following methods.

Method A

Compounds of formula I(a):

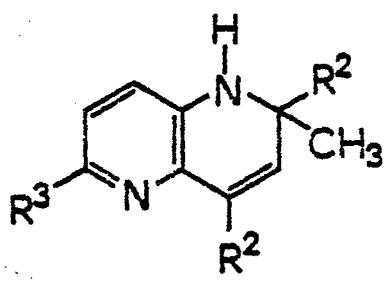

(Ia)

(in which $R^2$ and $R^3$ are as defined above) may be prepared by reacting a compound of formula (II):

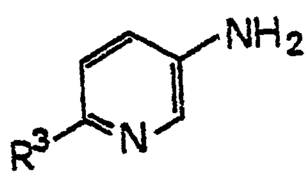

(II)

(in which $R^3$ is as defined above) with a compound of formula (III):

$$R^2COCH_3 \qquad (III)$$

(in which $R^2$ is as defined above).

The preferred compound of formula (III) is acetone.

This reaction is preferably effected in the presence of a catalytic amount of an acid (which may be a mineral acid, such as hydrochloric acid or sulphuric acid, or an organic acid, such as *p*-toluenesulphonic acid) or a catalytic amount of iodine. The compound of formula (III) normally serves as the reaction solvent.

The temperature at which the reaction is carried out is not particularly critical and we normally prefer to effect the reaction at a temperature within the range from 0°C to 150°C. The time required for the reaction will vary, depending upon the nature of the compounds employed and the reaction temperature, but usually from 1 to 50 hours suffices. After completion of the reaction, the reaction mixture may be treated by conventional means to recover the desired compound of formula I(a), which may, if necessary, be further purified by such conventional techniques as column chromatography, distillation or recrystallization.

Method B

Compounds of formula I(a) may also be prepared by reacting the aforementioned compound of formula (II) with a compound of formula (IV):

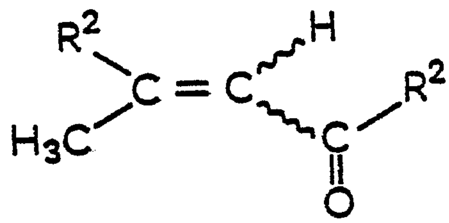

(IV)

(in which $R^2$ is as defined above). In formula (IV), the wavy lines mean, as is conventional, that the hydrogen atom and the carbonyl group attached by them to the doubly-bonded carbon atom may either be in the positions shown relative to the other substituents on the double bond or may be in the opposite positions, i.e. they may be cis or trans and the particular positions in which these substituents are drawn in the formula have no steric significance.

The preferred compound of formula (IV) is mesityl oxide.

This reaction is preferably effected in the presence of an acidic catalyst, which may be a mineral acid, such as hydrochloric acid or sulphuric acid, or an organic acid, such as p-toluenesulphonic acid. The reaction may be carried out without any solvent or in the presence of a suitable solvent, the nature of which is not critical, provided that it has no adverse effect upon the reaction. Suitable solvents include ethers (such as

diethyl ether or tetrahydrofuran) or halogenated hydrocarbons (such as methylene chloride or chloroform). The reaction temperature is not particularly critical, but we normally prefer to use a temperature within the range from 0°C to 150°C. The time required for the reaction will vary, depending upon the reagents and the reaction temperature, but a period of from 2 to 60 hours will usually suffice.

When the reaction is complete, the reaction mixture may be treated by conventional means to recover the desired compound of formula I(a) which may, if necessary, be further purified by such conventional techniques as column chromatography, recrystallization or distillation.

Method C

Compounds of formula I(a) may also be prepared by reacting the aforementioned compound of formula (II) with a compound of formula (V):

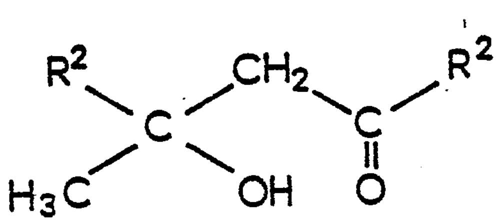

(V)

(in which $R^2$ is as defined above).

The preferred compound of formula (V) is diacetone alcohol.

The reaction is preferably effected in the presence of an acidic catalyst, which may be a mineral acid (such as hydrochloric acid or sulphuric acid) or an organic acid (such as *p*-toluenesulphonic acid). The reaction may be carried out without any solvent or in the presence of a suitable solvent, the nature of which is not critical, provided that it has no adverse effect upon the reaction. Suitable solvents include: aromatic hydrocarbons, such as benzene, toluene or xylene; aliphatic hydrocarbons, such as hexane or heptane; ethers, such as diethyl ether, tetrahydrofuran or dioxane; halogenated hydrocarbons, such as methylene chloride or chloroform; acetonitrile; or dimethylformamide.

The reaction temperature is not particularly critical and we normally prefer to carry out the reaction at a temperature within the range from 0°C to 150°C. The time required for the reaction will vary, depending upon the reagents used and the reaction temperature, but a period of from 2 to 60 hours will usually suffice.

Upon completion of the reaction, the reaction mixture may be treated by conventional means to recover the desired compound of formula I(a) which may then be further purified by conventional techniques, such as column chromatography, distillation or recrystallization, if desired.

Method D

Compounds of formula I(b):

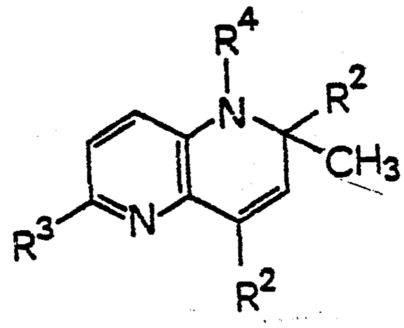

I(b)

(in which $R^2$, $R^3$ and $R^4$ are as defined above) may be prepared by reacting a compound of formula I(a), which have may been prepared by any of Methods A, B and C, with a compound of formula (VI):

$$R^4X \qquad (VI)$$

(wherein $R^4$ is as defined above and X represents a halogen atom or an acyloxy group, provided that X only represents an acyloxy group when $R^4$ represents an acyl group).

The reaction is preferably effected in the presence of a solvent, the nature of which is not critical, provided that it has no adverse effect upon the reaction. Suitable solvents include: halogenated hydrocarbons, such as methylene chloride, chloroform or carbon tetrachloride; alcohols, such as methanol, ethanol or t-butanol; acetonitrile; or dimethylformamide.

The reaction is preferably also effected in the presence of a base. Suitable bases include: alkali metal hydrides, such as lithium hydride or sodium hydride; alkyllithium compounds, such as butyllithium or sec-butyllithium; alkoxides, such as sodium methoxide, sodium ethoxide or potassium t-butoxide; tertiary amines, such as triethylamine or tributylamine; and heterocyclic bases, such as pyridine or picoline.

The temperature at which the reaction is carried out is not particularly critical, but we prefer to employ a temperature within the range from 10°C to 150°C. The time required for the reaction will vary, depending upon the reagents and the reaction temperature, but a period of from 1 to 20 hours will usually suffice.

Upon completion of the reaction, the reaction mixture may be treated by conventional means to recover the desired compound of formula I(b), which may then, if

necessary, be further purified by such conventional techniques as column chromatography, recrystallization or distillation.

Depending upon the particular reaction conditions, the compounds of formulae I(a) and I(b) prepared by Methods A - D may be obtained in the form of the free base or as an acid addition salt. Conversion of the resulting free base to the corresponding salt or conversion of the salt to the free base may be effected by conventional means.

Preferred pharmaceutically acceptable acid addition salts include salts with mineral acids (such as hydrochloric acid, phosphoric acid, sulphuric acid or nitric acid) and salts with organic acids (such as tartaric acid, citric acid, malic acid, lactic acid or maleic acid). Of these, the hydrochlorides are generally preferred.

The compounds of the invention have been tested for pharmacological activity and found to exhibit anti-inflammatory, analgesic and lipid peroxidation inhibitory activities. Details of certain of the pharmacological tests are as follows:

Carrageenin oedema test for anti-inflammatory activity

Male Wistar rats weighing 120-150 g were fasted overnight and then received a test compound per os as an aqueous tragacanth suspension. 30 minutes later, inflammation was induced by the subcutaneous injection of 0.05 ml of a 1% carrageenin suspension into the plantar tissue of a hind paw of each rat [Winter et al., Proc. Soc. Exp. Biol. Med., 111, 544 (1962)]. The anti-oedema activity was measured volumetrically, by assessing the response, as calculated from the following equation:

$$\text{Response} = (V - V_o)/V_o$$

where $V_o$ and V represent, respectively, the paw volume immediately before and 3 hours after the carrageenin injection. The test compounds were administered at a dose of 100 mg/kg and the results are reported in the following Table as the inhibitory rate, that is the percentage inhibition of the pain response.

Pain test for analgesic activity

This test was conducted according to a modification of the method reported by L.O. Randall and J.J. Selitto in Arch. Int. Pharmacodyn., 11, 409 (1959), proposed by Winter and Flatake (1957).

Male Wistar rats of 4 weeks of age and weighing 60-90 g were injected with 0.1 ml of a 20% by weight suspension of Brewers' yeast in the right hind paw. 4 hours later, rats which had a pain threshold to pressure-induced pain less than 10 x 30g were selected. Each of these was given orally a test compound as an aqueous tragacanth suspension at a dose of 50 mg/kg. 1 and 2 hours after administration of the test compound, the pain threshold was determined by observing pain responses (such as struggling or squeaking) when the inflamed or normal paw was subjected to pressure by a machine (Ugo-Basile). An "effective" animal was defined, in accordance with Blane's method (1968), as an animal which showed at least twice the mean pain threshold of control animals. The results are reported as the number of effective animals as a proportion of the total number of animals in each group.

The results are reported in the following Table 1, in which the compounds of the invention are identified by the numbers heretofore assigned to them.

TABLE 1

| Compound No. | Activity | |
|---|---|---|
| | Anti-inflammatory (%) | Analgesic |
| 1 | 62.6 | 3/5 |
| 2 | 58.2 | 1/5 |
| 4 | 65.4 | 3/5 |
| 5 | 53.6 | 3/5 |
| 13 | 56.6 | 3/5 |
| 18 | 76.0 | 4/5 |
| 25 | 54.8 | 2/5 |
| 27 | 53.3 | 1/5 |

The results given above show that the compounds have useful analgesic and anti-inflammatory activities, substantially more potent than, for example, phenylbutazone. The compounds of the invention are preferably administered in admixture with a carrier or diluent in the form of conventional pharmaceutical composition, preferably orally or rectally. Compositions for oral administration may be formulated as, for example, tablets, capsules, granules, powders or syrups, whilst compositions for rectal administration may be in the

form of suppositories . The compounds are also effective when applied topically, for example in the form of an ointment or a cream. The dosage employed will vary, depending upon the condition, age and body weight of the patient, but usually the dose for oral administration would be from 50 to 2000 mg per day for an adult, which may be administered in a single dose or in divided doses.

The preparation of the compounds of the invention is further illustrated by the following Examples.

## EXAMPLE 1

6-Methoxy-2,2,4-trimethyl-1,2-dihydro-1,5- naphthyridine (Compound No. 1).

100 ml of acetone were added to 50 g of 5-amino-2-methoxypyridine, and to the resulting mixture were added 5 ml of diethyl ether which had been saturated with hydrogen chloride gas. The mixture was then heated under reflux for 8 hours, after which it was cooled and the excess acetone was distilled off. The residue was subjected to column chromatography through silica gel eluted with a 9 : 1 by volume mixture of hexane and ethyl actate, giving the desired Compound No. 1, which was then purified by distillation, to give 45 g of an oily substance boiling at 108 - 111°C/200 Pa (1.5 mmHg).

Elemental analysis:

Calculated for $C_{12}H_{16}N_2O$:

C, 70.56%; H, 7.90%; N, 13.72%

Found: C, 70.32%; H, 8.12%; N, 13.67%

EXAMPLE 2

6-Chloro-2,2,4-trimethyl-1,2-dihydro-1,5-naphthyridine (Compound No. 13)

10 ml of acetone and 0.5 g of iodine were added to 17.3 g of 5-amino-2-chloropyridine, after which the mixture was heated under reflux for 13 hours. It was then cooled and the excess acetone was distilled off. The residue was subjected to column chromatography through silica gel eluted with a 9:1 by volume mixture of hexane and ethyl acetate, to give the desired Compound No.13. This was purified by recrystallisation from a mixture of diethyl ether and hexane, to give 2.5g of crystals melting at 75-76°C.

Elemental Analysis:

Calculated for $C_{11}H_{13}N_2Cl$:

C, 63.30%; H, 6.27%; N,13.42; Cl, 16.98%.

Found: C,63.40%; H,6.30%; N, 13.29%; Cl, 16.77%.

EXAMPLES 3-36

Following the procedure described in Example 1, but employing different pyridine derivatives as starting materials, there were prepared the compounds shown in the following Table 2. In this Table, the compounds are identified by the numbers assigned to them in the foregoing list and the abbreviations "mp." and "bp." mean "melting point" and "boiling point", respectively, in degrees Celsius. The boiling points are reported as the temperature of boiling, followed by the pressure (in Pascals) at which boiling took place. The compounds were obtained either as the free base or as the hydrochloride and, in some cases, the hydrochloride was converted to the free base or vice versa.

TABLE 2

| Compound No. | mp. or bp. free base | mp. of hydrochloride |
|---|---|---|
| 2 | - | 162 - 165 |
| 4 | bp 135/53 | - |
| 5 | bp 170 - 175/80 | 179 - 181 |
| 6 | mp 70 - 72 | - |
| 7 | mp 70 - 72 | - |
| 8 | bp 145 - 150/80 | 160 - 163 |
| 9 | bp 165 - 170/40 | 163 - 169 |
| 10 | mp 102 - 104 | - |
| 14 | bp 170 - 175/67 | - |
| 15 | mp 68 - 69 | - |
| 16 | - | 150 - 153 |
| 17 | bp 173 - 178/67 | - |
| 18 | - | 176 - 181 |
| 19 | mp 130 - 134 | - |
| 20 | - | 117 - 118* |
| 23 | bp 175 - 180/53 | - |
| 34 | bp 183 - 188/53 | - |
| 36 | mp 108 - 109 | - |
| 37 | - | 157 - 160 |
| 38 | bp 180 - 185/27 | - |
| 39 | mp 148 - 150 | - |
| 40 | - | 122 - 125 |

TABLE 2 - (CONTINUED)

| Compound No. | mp. or bp. free base | mp. of hydrochloride |
|---|---|---|
| 41 | - | 113 - 117 |
| 42 | bp 180 - 185/93 | 162 - 164 |
| 43 | bp 210/67 | 151 - 154 |
| 44 | - | 146 - 150 |
| 45 | - | 173 - 177 |
| 46 | mp 60 - 62 | - |
| 51 | bp 212 - 215/53 | - |
| 52 | bp 210 - 215/40 | 148 - 153 |
| 53 | - | 158 - 161 |
| 54 | mp 95 - 96 | - |
| 55 | - | 167 - 170 |
| 56 | - | 167 - 171 |

* Compound No.20 was converted to the 1-acetyl derivative, prior to measurement of the melting point of the hydrochloride thereof.

EXAMPLE 37

6-Methoxy-1,2,2,4-tetramethyl-1,2-dihydro-1,5-naphthyridine (Compound No 25)

6.2 g of 6-methoxy-2,2,4-trimethyl-1,2-dihydro-1,5-

naphthyridine (Compound No. 1) were dissolved in 40 ml of dimethylformamide, and 1.5 g of sodium hydride were added to the resulting solution, which was then stirred for 30 minutes. To this mixture were then added dropwise 8 g of methyl iodide in dimethylformamide, and the mixture was stirred at room temperature for 48 hours. It was then poured into ice-water and extracted with ethyl acetate. The solvent was distilled off and the residue was subjected to column chromatography through silica gel eluted with a 9:1 by volume mixture of hexane and ethyl acetate, to give the desired Compound No. 25. This was purified by distillation, giving 4.2 g of an oily substance boiling at 130-135°C/67 Pa (0.5 mm Hg).

Elemental analysis:

Calculated for $C_{13}H_{18}N_2O$:

| | |
|---|---|
| | C, 71.52%; H, 8.31%; N, 12.83%. |
| Found: | C, 71.49%; H, 8.37%; N, 12.74%. |

## EXAMPLE 38

### 6-Butoxy-1,2,2,4-tetramethyl-1,2-dihydro-1,5-naphthyridine (Compound No 47)

28 g of 5-amino-2-butoxypyridine and 1 ml of diethyl ether which had been saturated with hydrogen

chloride gas were added to 50 ml of acetone, and the mixture was heated under reflux for 27 hours. It was then cooled and the excess acetone was distilled off. The residue was subjected to column chromatography through silica gel eluted with benzene, giving 18 g of 6-butoxy-2,2,4- trimethyl-1,2-dihydro-1,5-naphthyridine (Compound No 4). To 2.5 g of this Compound No. 4 were added 30 ml of acetone and 2.7 g of methyl iodide, and the mixture was heated under reflux for 10 hours. It was then cooled and the acetone was distilled off. The residue was subjected to column chromatography through silica gel eluted with a 9:1 by volume mixture of hexane and ethyl acetate, to give 0.85 g of the desired Compound No. 47, as an oily substance.

Elemental analysis:
Calculated for $C_{16}H_{24}N_2O$:

| | |
|---|---|
| | C, 73.80%; H, 9.29%; N, 10.76%. |
| Found: | C, 73.44%; H, 9.33%; N, 10.34%. |

## EXAMPLE 39

### 1-(p-Chlorobenzoyl)-6-methoxy-2,2,4-trimethyl-1,2-dihydro-1,5-naphthyridine (Compound No 48)

3 g of 6-methoxy-2,2,4-trimethyl-1,2-dihydro-1,5-naphthyridine (Compound No. 1) were dissolved in 15 ml

of dimethylformamide. 0.8 g of sodium hydride was added to the resulting solution, which was then stirred for 30 minutes, after which 5.6 g of p-chlorobenzoyl chloride dissolved in dimethylformamide were added dropwise. The mixture was then heated at 60°C for 10 hours, after which it was poured into ice-water and extracted with ethyl acetate. The solvent was distilled from the extract and the residue was subjected to column chromatography through silica gel eluted with benzene, to give the desired Compound No. 48. This was purified by recrystallization from diethyl ether, giving 2.0 g of crystals melting at 79-82°C.

Elemental analysis:

Calculated for $C_{19}H_{19}N_2O_2Cl$:

C, 66.56%; H, 5.58%; N, 8.17%; Cl, 10.34%.

Found: C, 66.71%; H, 5.53%; N, 8.19%; Cl, 10.40%.

## EXAMPLE 40

1-(o-Fluorobenzoyl)-6-methoxy-2,2,4-trimethyl-1,2-dihydro-1,5-naphthyridine (Compound No 49)

3 g of 6-methoxy-2,2,4-trimethyl-1,2-dihydro-1,5-naphthyridine (Compound No. 1) were dissolved in 20 ml

of dimethylformamide. 0.8 g of sodium hydride were added to the mixture, which was then stirred for 30 minutes. After this, 5.0 g of o-fluorobenzoyl chloride dissolved in dimethylformamide were added dropwise to the mixture, which was then heated at 60°C for 10 hours. The mixture was then poured into ice-water and extracted with ethyl acetate. The solvent was distilled from the extract and the residue was subjected to column chromatography through silica gel eluted with a 9:1 by volume mixture of hexane and ethyl acetate, to give the desired Compound No. 49. This was purified by recrystallization from a mixture of diethyl ether and hexane, to give 1.5 g of crystals melting at 102-104°C.

Elemental analysis:

Calculated for $C_{19}H_{19}N_2O_2F$:

C, 69.92%; H, 5.86%; N, 8.58%; F, 5.82%.

Found: C, 69.94%; H, 5.80%; N, 8.60%; F, 5.60%.

EXAMPLE 41

1-Acetyl-6-methoxy-2,2,4-trimethyl-1,2-dihydro-1,5-naphthyridine (Compound No 27)

5 g of acetic anhydride were added to 3 g of 6-methoxy-2,2,4-trimethyl-1,2-dihydro-1,5-naphthyridine,

after which the mixture was heated under reflux for 3 hours. It was then cooled and extracted with ethyl acetate. The solvent was distilled from the extract and the residue was subjected to column chromatography through silica gel eluted with a 9:1 by volume mixture of hexane and ethyl acetate, to give the desired Compound No. 27. This was purified by recrystallization from diethyl ether, giving 2.6 g of crystals melting at 61-63°C.

Elemental analysis:

Calculated for $C_{14}H_{18}N_2O_2$:

C, 68.27%; H, 7.37%; N, 11.37%.

Found: C, 68.37%; H, 7.30%; N, 11.48%.

## EXAMPLE 42

1-Acetyl-2,2,4-trimethyl-6-morpholino-1,2-dihydro-1,5-naphthyridine (Compound No 50)

35 ml of acetone and 0.1 g of iodine were added to 4.7 g of 5-amino-2-morpholinopyridine, and the mixture was heated under reflux for 15 hours. It was then cooled and the excess acetone was distilled off. The residue was subjected to column chromatography through silica gel eluted with a 8:2 by volume mixture of hexane and ethyl acetate, to give 2.4 g of 2,2,4-trimethyl-6-morpholino-1,2-dihydro-1,5-naphthyridine (Compound No. 19), melting at 137-141°C. To this Compound No. 19 were added 20 ml of acetic anhydride, and the mixture was heated under reflux for 3 hours. It was then cooled and the mixture was extracted with ethyl acetate. The solvent was distilled from the extract and the residue was recrystallized from diethyl ether, to give 2.8 g of the desired Compound No. 50, melting at 108-110°C.

Elemental analysis:

Calculated for $C_{17}H_{23}N_3O_2$:

| | |
|---|---|
| | C, 67.75%; H, 7.69%; N, 13.94%. |
| Found: | C, 67.85%; H, 7.69%; N, 13.91%. |

EXAMPLE 43

2,4-Diethyl-6-methoxy-2-methyl-1,2-dihydro-1,5-naphthyridine (Compound No 30)

To 25 g of 5-amino-2-methoxypyridine were added 30 g of methyl ethyl ketone, followed by 2 ml of diethyl ether saturated with hydrogen chloride gas. The mixture was heated under reflux for 20 hours and then cooled. The excess methyl ethyl ketone was distilled off and the residue was subjected to column chromatography through silica gel eluted with a 9:1 by volume mixture of hexane and ethyl acetate, to give the desired Compound No. 30.

This was purified by distillation, giving 5.2 g of an oily substance boiling at 125-130°C/40 Pa (0.3 mm Hg).

Elemental analysis:

Calculated for $C_{14}H_{20}N_2O$:

C, 72.38%; H, 8.68%; N, 12.06%.

Found: C, 72.00%; H, 8.68%; N, 11.73%.

## EXAMPLE 44

### 6-Methoxy-2,2,4-trimethyl-1,2-dihydro-1,5-naphthyridine (Compound No 1)

1.96 g of mesityl oxide and a drop of concentrated hydrochloric acid were added to 2.48 g of 5-amino-2-methoxypyridine. The mixture was left to stand for 2 days and then subjected to column chromatography through silica gel eluted with 9:1 by volume mixture of benzene and hexane, to give 2.5 g of the desired Compound No. 1 having the same properties and analysis as the product of Example 1.

## EXAMPLE 45

6-Methoxy-2,2,4-trimethyl-1,2-dihydro-1,5-naphthyridine (Compound No 1)

2.40 g of diacetone alcohol and a drop of concentrated hydrochloric acid were added to 2.48 g of 5-amino-2-methoxypyridine. The mixture was left to stand for 4 days and then subjected to column chromatography through silica gel eluted with benzene, to give 2.1 g of the desired Compound No. 1, having the same properties and analysis as the product of Example 1.

CLAIMS:

1. Compounds of formula (I):

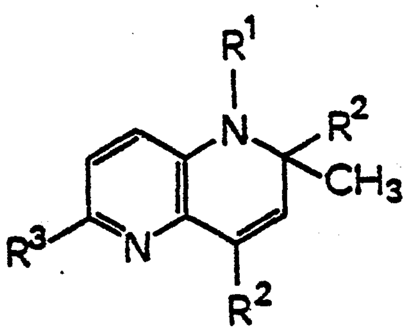

(I)

(in which:
$R^1$ represents a hydrogen atom, an alkyl group or an acyl group;
$R^2$ represents an alkyl group and the two groups represented by $R^2$ may be the same or different; and
$R^3$ represents a hydrogen atom, a halogen atom, an alkyl group, a hydroxy group, an alkoxy group, an aryloxy group, a pyridyloxy group, a pyridyloxy group having at least one substituent in its pyridine ring, an alkylthio group, an arylthio group, an amino group or a mono- or di-substituted amino group),
and pharmaceutically acceptable acid addition salts thereof.

2. Compounds as claimed in claim 1, wherein:
$R^1$ represents a hydrogen atom, an alkyl group having from 1 to 6 carbon atoms, an aliphatic acyl group having from 1 to 4 carbon atoms, an aromatic acyl group or an araliphatic acyl group;

the groups represented by $R^2$ are each alkyl groups having from 1 to 3 carbon atoms; and $R^3$ represents a hydrogen atom, a halogen atom, a $C_1$-$C_3$ alkyl group, a $C_1$-$C_3$ alkoxy group, a phenoxy, phenylthio or pyridyloxy group which is unsubstituted or has one or more halogen, $C_1$-$C_6$ alkyl, $C_1$-$C_6$ alkoxy, di($C_1$-$C_6$ alkyl)amino, carboxy, ($C_1$-$C_6$alkoxy)-carbonyl or ($C_1$-$C_6$ alkoxy)carbonyl($C_1$-$C_6$ alkyl) substituents, a $C_1$-$C_3$ alkylthio group, an amino group, a group of formula $NHR^5$ (wherein $R^5$ represents a $C_1$-$C_6$ alkyl group, or a phenyl or pyridyl group which is unsubstituted or has one or more substituents on its aromatic ring) or a group of formula $-NR^6R^7$ (wherein $R^6$ and $R^7$ are the same or different and each represents an alkyl group or, together with the nitrogen atom to which they are attached, form a heterocyclic amino group).

3. Compounds as claimed in claim 1 or claim 2, wherein the two groups represented by $R^2$ are identical and are each $C_1$-$C_3$ alkyl groups.

4. Compounds as claimed in claim 3, wherein $R^2$ represents a methyl group.

5. The following compounds as claimed in claim 1:

6-methoxy-2,2,4-trimethyl-1,2-dihydro-1,5-naphthyridine;
6-ethoxy-2,2,4-trimethyl-1,2-dihydro-1,5-naphthyridine;
6-butoxy-2,2,4-trimethyl-1,2-dihydro-1,5-naphthyridine;
2,2,4-trimethyl-6-phenoxy-1,2-dihydro-1,5-naphthyridine;
6-chloro-2,2,4-trimethyl-1,2-dihydro-1,5-naphthyridine;
2,2,4-trimethyl-6-(3-pyridyloxy)-1,2-dihydro-1,5-naphthyridine;
6-methoxy-1,2,2,4-tetramethyl-1,2-dihydro-1,5-naphthyridine; and
1-acetyl-6-methoxy-2,2,4-trimethyl-1,2-dihydro-1,5-naphthyridine.

6. A process for preparing a compound as claimed in any one of the preceding claims, which process comprises the steps:

(a) reacting a compound of formula (II):

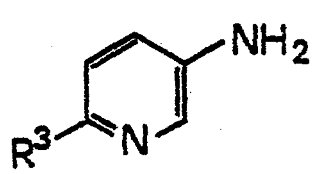

(II)

(wherein $R^3$ is as defined in claim 1) with a compound of formula (III), (IV) or (V):

$R^2COCH_3$ (III)

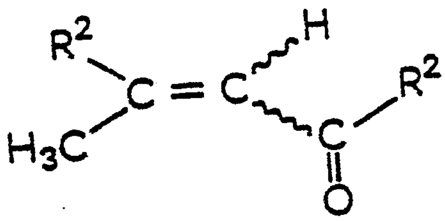

(IV)

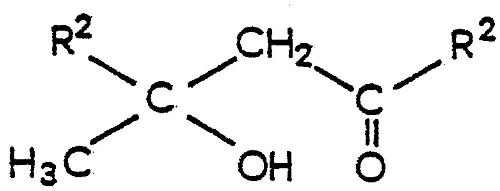

(V)

(wherein $R^2$ is as defined in claim 1), to give a compound of formula I(a):

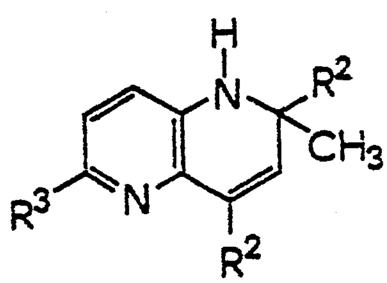

(Ia)

(wherein $R^2$ and $R^3$ are as defined in claim 1);

(b) optionally, reacting said compound of formula I(a) with a compound of formula (VI):

$R^4X$ (VI)

(in which:

$R^4$ represents an alkyl group and X represents a halogen atom; or

$R^4$ represents an acyl group and X represents a halogen atom or an acyloxy group), to give a compound of formula I(b):

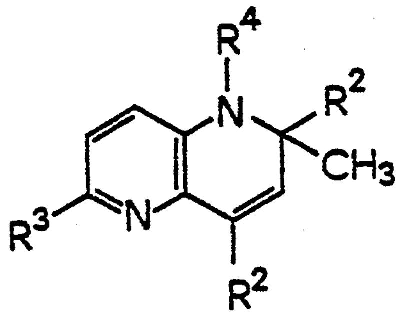

(Ib)

(wherein $R^2$, $R^3$ and $R^4$ are as defined above); and

(c) optionally salifying the product of step (a) or (b).

7. A process as claimed in claim 6, wherein said compound of formula (III) is acetone.

8. A process as claimed in claim 6, wherein said compound of formula (IV) is mesityl oxide.

9. A process as claimed in claim 6, wherein said compound of formula (V) is diacetone alcohol.

10. A pharmaceutical composition for analgesic or anti-inflammatory use and comprising an analgesic and

anti-inflammatory agent in admixture with a pharmaceutically acceptable carrier or diluent, wherein said agent is a compound as claimed in any one of claims 1 to 6 or as prepared by a process claimed in any one of claims 7 to 9.